(19) Europäisches Patentamt
European Patent Office
Office européen des brevets

(11) EP 2 260 870 A2

(12) **EUROPEAN PATENT APPLICATION**

(43) Date of publication:
**15.12.2010 Bulletin 2010/50**

(51) Int Cl.:
***A61K 45/06*** *(2006.01)* ***A61K 31/352*** *(2006.01)*
***A61P 31/00*** *(2006.01)* ***A61P 33/00*** *(2006.01)*

(21) Application number: **10181710.4**

(22) Date of filing: **29.04.2005**

(84) Designated Contracting States:
**AT BE BG CH CY CZ DE DK EE ES FI FR GB GR HU IE IS IT LI LT LU MC NL PL PT RO SE SI SK TR**

(30) Priority: **30.04.2004 DK 200400680**
**08.11.2004 DK 200401716**

(62) Document number(s) of the earlier application(s) in accordance with Art. 76 EPC:
**05736144.6 / 1 744 781**

(71) Applicant: **BKG Pharma ApS**
**2920 Charlottenlund (DK)**

(72) Inventor: **Giwercman, Birgit Kjældgaard**
**2920, Charlottenlund (DK)**

(74) Representative: **Bender, Mikkel et al**
**Chas. Hude A/S**
**H.C. Andersens Boulevard 33**
**DK-1780 Copenhagen V (DK)**

Remarks:
This application was filed on 29-09-2010 as a divisional application to the application mentioned under INID code 62.

(54) **Treatment of infectious diseases with combinations of a thioxanthene derivative with an anti-infective agent**

(57) The present invention is directed to chemosensitising compounds, in particular thioxanthene derivatives, for treatment of infectious diseases in combination with an anti-infective agent. The invention furthermore relates to compositions comprising said chemosensitising compounds and anti-infective agents.

**FIG.1**

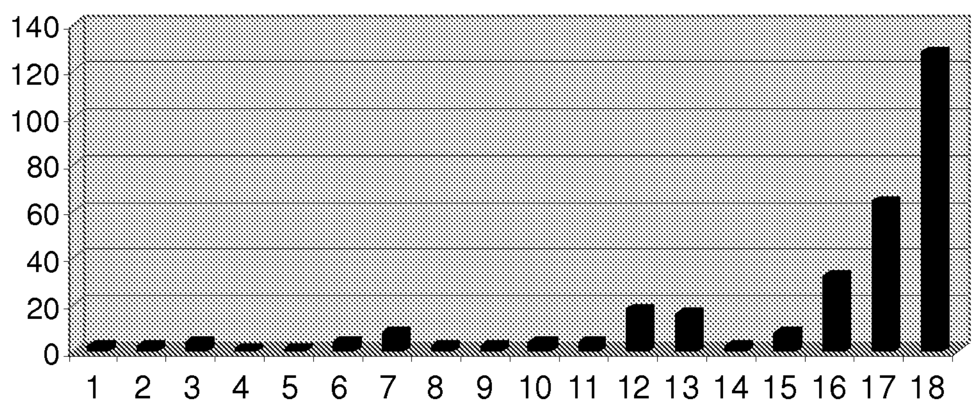


EP 2 260 870 A2

## Description

### FIELD OF THE INVENTION

The present invention is directed to the use of chemosensitising compounds, in particular thioxanthene derivatives and phenothiazine derivatives, for treatment of infectious diseases in combination with an anti-infective agent.

### BACKGROUND OF THE INVENTION

Resistance to chemotherapy is a common clinical problem in patients with infectious diseases. During treatment of infections the drug targets of prokaryotic or eukaryotic microorganisms cells are often found to be refractory to a variety of drugs that have different structures and functions. This phenomenon has been termed multidrug resistance (MDR). Organisms ranging from bacteria to human beings possess transmembrane transporters which confer resistance to toxic compounds. Underlining their biological significance, prokaryotic and eukaryotic multidrug transport proteins are very similar in structure and function. In addition, other kinds of important resistance mechanisms in combination with efflux pumps may co-operate thereby giving rise to high levels of resistance. There is an urgent need for drugs that can inhibit (reverse) or circumvent the resistance mechanisms and improve the effectiveness of commonly used anti-infective agents.

The incidence of the multiple antimicrobial resistance of bacteria which cause infections in hospitals/intensive care units is increasing, and finding microorganisms insensitive to more than 10 different antibiotics is not unusual. Examples of such resistant bacteria include methicillin-resistant and methicillin-vancomycin-resistant *Staphylococcus aureus*; vancomycin-resistant enterococci, such as *Enterococcus faecalis* and *Enterococcus faecium*; penicillin-resistant *Streptococcus pneumoniae*, and cephalosporin and quinolone resistant gram-negative rods (coliforms), such as *E. coli, Salmonella* species, *Klebsiella pneumoniae, Pseudomonas* species and *Enterobacter* species. More recently, pan antibiotic resistant gram-negative and gram-positive bacilli have emerged.

The rapidity of emergence of these multiple antibiotic-resistance is not being reflected by the same rate of development of new antibiotics and it is, therefore, conceivable that patients with serious infections soon will no longer be treatable with currently available anti-infective agents. Several international reports have highlighted the potential problems associated with the emergence of antimicrobial resistance in many areas of medicine and also outlined the difficulties in the management of patients with infections caused by these microorganisms.

Although most of the hardier microorganisms are present in hospitals, strains of multidrug resistant bacteria, such as *Streptococcus pneumoniae* and *Mycobacterium tuberculosis* have also caused serious community-acquired infections. The prevalence of drug-resistant *Streptococcus pneumoniae* has increased 60-fold since 1980 with 51% and 8% of isolates demonstrating intermediate- or high-level resistance to penicillin or third-generation cephalosporins, respectively. Thus, pneumococcal pneumonia is becoming more difficult to treat with first-line anti-infective agents. Resistant bacteria from hospitals can be introduced into the community via patients discharged for continued treatment at home taking with them, for example, multidrug resistant *Staphylococcus aureus* and vancomycin resistant enterococci.

Mechanisms of resistance in bacteria can be mediated either via chromosomes or via plasmids. Studies of emerging resistance show that resistance in bacteria can arise in steps progressing from low level to high level, unless a plasmid is acquired on which full-blown resistance is already present. For example, the initial penicillin resistant pneumococci appeared with slightly decreased susceptibility to the antibiotic but over time evolved high-level resistance. Penicillin and tetracycline resistance among gonococci emerges in a similar way. The phenomenon has also been observed with pan resistant *E. coli,* where multiple steps are required to reach a clinically relevant level of resistance.

Anti-infective agents may be rendered inactive by three major mechanisms: i) destruction or modification of the antibiotic (for example, by production of beta-lactamases and aminoglycoside-inactivating enzymes), ii) alteration of the target site, and iii) prevention of access to the target (for example, alteration of permeability or efflux).

All of the three major resistance mechanisms alone ore in combination are clinical important, leading to treatment failure. However, recently efflux-related multidrug resistance (MDR) has become appreciated as a significant complicating factor in the chemotherapy of bacterial infections.

Efflux mechanisms that account for resistance to a variety of anti-infective agents are commonly found in a wide range of bacteria. The term MDR system refers to a group of transporters, which are able to expulse a wide range of quite different substrates. While this type of system was first described in eukaryotic cells in the late 1980s, the presence of MDR efflux pumps in bacteria showing resistance to several drugs has been increasingly reported in the literature. MDR cell lines in general are associated with decreased drug accumulation due to enhanced efflux as well as diminished influx of chemotherapeutic drugs.

Over expression of MDR efflux pumps, after induction or because of the emergence of mutations in their regulatory elements, is a major mechanism in acquired bacterial resistance to multiple anti-infective agents. Two major groups of efflux systems are known, specific exporters and transporters conferring multidrug resistance (MDR). The

MDR systems are able to remove anti-infective agents of different classes from the bacterial cell and occasionally play a role in the intrinsic resistance of some bacteria to certain anti-infective agents. Their genes are commonly located on the bacterial chromosome. The mechanisms leading to MDR are frequently caused by transmembrane xenobiotic transport molecules belonging to the superfamily of ATP-binding cassette (ABC) transporters. MDR efflux has mainly been identified with resistance to compounds such as tetracyclines, fluoroquinolones, macrolides, lincosamides rifampicin, chlorphenicol, and aminoglycosides. In contrast, the genes coding for specific efflux systems are often associated with mobile genetic elements, which can easily be interchanged between bacteria. Specific efflux systems have mainly been identified with resistance to compounds such as macrolides, lincosamides and/or streptogramins, tetracyclines, as well as chloramphenicol in Gram positive and Gram negative bacteria.

Prokaryotic and eukaryotic multidrug transport proteins are very similar in structure and function. For eukaryotic cells, drug efflux pumps have been viewed by many authors as complementing enzyme-based detoxification systems.

In prokaryotic cells, several kinds of resistance mechanisms in combination with efflux pumps may co-operate, resulting in high levels of resistance and therapeutic failure.

Inhibition of resistance mechanisms can restore the activities of anti-infective agents that are substrates for these mechanisms. The recent demonstration and elucidation of the phenomenon of developing resistance has led to the search for drugs that could improve the effectiveness of anti-infective agents.

Two potential groups of MDR inhibitors are the phenothiazines and thioxanthenes. Phenothiazines and thioxanthenes are used clinically as neuroleptic and antiemetic agents. Phenothiazines, and structurally related antipsychotic agents, inhibit several cellular enzymes and block the function of critical cellular receptors. The extrapyramidal side effects associated with antipsychotic therapy are attributed to dopamine receptor binding. In general these extrapyramidal side effects have proven to be dose limiting in clinical trials using phenothiazines and thioxanthenes in non-psychotic areas, such as anti-cancer treatment.

Phenothiazines and thioxanthenes have been shown to inhibit the function of eukaryotic MDR efflux pumps and certain prokaryotic MDR efflux pumps.

Phenothiazines have been shown to be among the group of drugs known to modify resistance to one or more antibacterial agents in certain bacteria. Although the mechanism by which phenothiazines and other drugs modulate MDR is not yet clear, it has been suggested that their pharmacological properties may be mediated at least in part by inhibition of efflux pumps. Also, promethazine has been recognised as an effective antiplasmid agent in cultures containing bacterial species such as *Escherichia coli, Yersinia enterocolitica, Staphylococcus aureus and Agrobacterium tumefaciens.* The concentrations used, however, are generally high above clinically relevant concentrations.

The fact that effective concentrations are above clinically relevant concentrations was emphasised by Kaatz et al. (2003). Although inhibition of efflux pumps in certain types of *S. aureus* by selected phenothiazines and the two geometric stereoisomers *cis*- and *trans*- flupenthixol were shown, the authors still concluded that "Unfortunately, the $IC_{50}$ values of inhibitors for EtBr, acriflavine and pyronin Y efflux are above those employed in clinical practice".

Phenothiazines and thioxanthenes have modest, but broad, antimicrobial activities. MICs are generally above clinically relevant concentrations inasmuch as the minimum effective concentrations *in vitro* are in the order from approximately 20 mg/l to several hundreds mg/l and the relevant serum levels range from approximately 0.3 μg/l to 0.5 mg/l (0.3 ng/ml to 0.5 μg/ml).

The thioxanthenes demonstrate geometric stereoisomerism. The *cis* and *trans* forms have previously been shown to have roughly equal modest antibacterial potency. MICs are generally far above clinically relevant concentrations. In 1998 Kristiansen et al. suggested a synergistic effect between penicillin and *trans*-clopenpenthixol in a study using a non-resistant mouse pathogen isolate of Streptococcus pneumoniae (MIC 0.02 μg/ml penicillin). However, the authors did not show significant differences of MIC values using penicillin alone and penicillin in combination with *trans*-clopenthixol, and concentration/response/ time correlation studies were not performed, i.e. the concentration of drugs in the infected mice were unknown. Also the response of the infecting bacteria in the mice were unknown.

Johnson et al. showed in a clinical trial of the anti-psychotic effect of cis-flupenthixol versus trans-flupenthixol versus placebo, that while *cis*-flupenthixol was a potent neuroleptic (especially for "positive" symptoms), trans-flupenthixol had no activity as an anti-psychotic. Since trans-flupenthixol is a far less potent dopamine antagonist, and the extrapyramidal side effects associated with antipsychotic therapy are attributed to dopamine receptor binding, trans-flupenthixol lacks these side effects.

The apparent lack of anti-psychotic activity or extrapyramidal side effects of the *trans*forms such as trans-flupenthixol and trans-clopenthixol makes them particularly attractive for use as anti-resistance agents.

Several phenothiazine and thioxanthene derivatives are disclosed in US 6,569,853.

Flupenthixol is disclosed in UK Patent 925,538 as having utility as tranquilliser, ataractic, antiemetic, antihistamine, antispasmodic and general central nervous system depressant. No mention is made of any anti-infective or anti-resistance activity.

Several thioxanthene derivatives are disclosed in UK Patent 863,699 as tranquillisers. No mention is made of any of anti-infective or anti-resistance activity.

From the discussion above it is clear that the increase in resistance to anti-infective agents, such as antibiotics, present a major impediment to the treatment of infections. Thus, there is an urgent need for drugs that can inhibit or circumvent the resistance mechanisms and improve the effectiveness of the currently available anti-infective agents.

The object of the present invention is to provide chemosensitising compounds capable of sensitising resistant, including multidrug resistant, cells or microorganisms to an anti-infective agent by administration of clinically relevant amounts of such chemosensitising compounds to a subject in need thereof. Another, but related, object of the invention is improve the effectiveness of anti-infective agents for treatment of infectious diseases, in particular when such infectious diseases are caused by resistant, including multidrug resistant, microorganisms.

## SUMMARY OF THE INVENTION

As will be understood from the above discussion, the prior art has hitherto deemed thioxanthenes and phenothiazines unsuitable for treatment of infectious diseases in combination with anti-infective agents, since the necessary therapeutic amount of such chemosensitising compounds would cause severe side effects.

The present inventor realised that the above conclusions in general were based on studies carried out on artificial resistant or multidrug resistant microorganisms or *in vitro* selected microorganisms. Thus, despite the prejudice in the art that thioxanthene and phenothiazine concentrations far above the clinical relevant level would be necessary in order to combat resistant or multidrug resistant microorganisms, the present inventor decided to investigate this matter in further detail by studying the effect of such chemosensitising compounds in combination with anti-infective agents on clinically relevant resistant and multidrug resistant isolates using clinically relevant amounts of the chemosensitising compounds described herein.

Surprisingly, it was found that by applying clinically relevant amounts of the chemosensitising compounds described herein in combination with anti-infective agents, effective killing of resistant and multidrug resistant clinically relevant isolates was achieved. Contrary to what was previously believed, this surprising finding opens up the possibility to effectively combat resistant and multidrug resistant microorganisms by a combination of the chemosensitising compounds described herein and commonly used anti-infective agents.

Accordingly, in a first aspect the present invention relates to the use of a compound of the general formula (I)

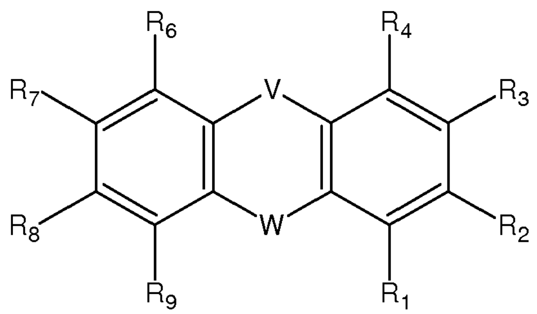

(I)

wherein

V is selected from the group consisting of S, $SO_2$, SO, O and NH;

W is $N-(CHX)_nN(R_{10})(R_{11})$ or W is $C=CH-(CHX)_m-N(R_{10})(R_{11})$;

n is an integer in the range of from 2 to 6;

m is an integer in the range of from 1 to 5;

each X is individually selected from the group consisting of hydrogen, halogen, hydroxy, amino, nitro, optionally substituted $C_{1-6}$-alkyl and optionally substituted $C_{1-6}$-alkoxy;

$R_1$, $R_2$, $R_3$, $R_4$, $R_5$, $R_6$, $R_7$, $R_8$ and $R_9$ are each individually selected from the group consisting of hydrogen, halogen, hydroxy, amino, nitro, optionally substituted $C_{1-6}$-alkyl, optionally substituted $C_{2-6}$-alkenyl, optionally substituted $C_{2-6}$-alkynyl and optionally substituted $C_{1-6}$-alkoxy, optionally substituted $C_{2-6}$-alkenyloxy, carboxy, optionally substituted $C_{1-6}$-alkoxycarbonyl, optionally substituted $C_{1-6}$-alkylcarbonyl, fomyl, optionally substituted $C_{1-6}$-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally

substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, mono- and di($C_{1-6}$-alkyl)amino, carbamoyl, mono- and di ($C_{1-6}$-alkyl)aminocarbonyl, amino-$C_{1-6}$-alkyl-aminocarbonyl, mono- and di($C_{1-6}$-alkyl)amino-$C_{1-6}$-alkylaminocarbonyl, $C_{1-6}$-alkylcarbonylamino, amino-$C_{1-6}$-alkyl-carbonylamino, mono- and di($C_{1-6}$-alkyl)amino-$C_{1-6}$-alkyl-carbonylamino, amino-$C_{1-6}$-alkyl-amino, mono- and di($C_{1-6}$-alkyl)amino-$C_{1-6}$-alkyl-amino, cyano, guanidino, carbamido, $C_{1-6}$-alkanoyloxy, $C_{1-6}$-alkylsulphonyl, $C_{1-6}$-alkylsulphinyl, $C_{1-6}$-alkylsulphonyloxy, aminosulfonyl, mono- and di($C_{1-6}$-alkyl)aminosulfonyl, and optionally substituted $C_{1-6}$-alkylthio; and

$R_{10}$ and $R_{11}$ are each independently selected from the group consisting of hydrogen, optionally substituted $C_{1-6}$-alkyl, optionally substituted $C_{2-6}$-alkenyl, optionally substituted $C_{2-6}$-alkynyl, optionally substituted $C_{1-6}$-alkoxycarbonyl, optionally substituted $C_{1-6}$-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di($C_{1-6}$-alkyl)aminocarbonyl; or $R_{10}$ and $R_{11}$ together with the nitrogen atom to which they are attached form an optionally substituted nitrogen-containing heteroaryl or optionally substituted heterocyclyl;

or a metabolite or salt thereof for the manufacture of a medicament for the treatment or prophylaxis of an infectious disease in combination with an anti-infective agent.

Other aspect of the present invention will be apparent from the below description and the appended claims.

## BRIEF DESCRIPTION OF THE FIGURES

Figure 1 shows a series of bars depicting the effect of chemosensitising compounds against drug resistant and multidrug resistant microorganisms (values from Tables 1-4 below). The Y-axis represents the DR Ratio and is defined as the ratio of the MIC for anti-infective agent alone divided by the MIC for the anti-infective agent in the presence of chemosensitising compound. This ratio represents the apparent increase in potency of the anti-infective agent produced by the individual chemosensitising compounds. Anti-infective agent: Coprofloxacin. Chemosensitising compounds: 1. promazine; 2. 1-chlorpromazine; 3. chlorpromazine; 4. 7-hydroxychlorpromazine; 5. 7,8-dihydroxychlorpromazine; 6. thiomethylpromazine; 7. trifluopromazine; 8. chlorpromazine sulfoxide; 9. desmethylchlorpromazine; 10. perphenazine; 11. prochlorperazine; 12. fluphenazine; 13. trifluoperazine; 14. 2-chloro-1 0-(2-dimethylaminoethyl)phenothiazine; 15. promethazine; 16. cis-flupenthixol; 17. *trans*-flupenthixol; 18. *trans*-clopenthixol.

Figures 2A and 2B show a series of bars depicting the strong synergistic effect of *cis*-flupenthixol, *trans*-flupenthixol and *trans*-clopenthixol on ciprofloxacin, gentamycin and piperacillin, respectively (values from Table 5 below). The tested microorganisms were *E. coli* 331 ME (Figure 2A) and *P. aeruginosa* 432b (Figure 2B). The Y-axis represents the Fractional Inhibitory Concentration (FIC) index. Synergy was defined for FIC indices less than 0.5.

## DETAILED DESCRIPTION OF THE INVENTION

### Definitions

In the present context, the term "$C_{1-6}$-alkyl" is intended to mean a linear or branched saturated hydrocarbon group having from one to six carbon atoms, such as methyl, ethyl, n-propyl, isopropyl, n-butyl, isobutyl, sec-butyl, *tert*-butyl, n-pentyl, isopentyl, neopentyl and n-hexyl.

In the present context the term "$C_{3-6}$-cycloalkyl" is intended to cover three-, four-, five-and six-membered rings comprising carbon atoms only, whereas the term "heterocyclyl" is intended to mean three-, four-, five- and six-membered rings wherein carbon atoms together with from 1 to 3 heteroatoms constitute said ring. The heteroatoms are independently selected from oxygen, sulphur, and nitrogen. $C_{3-6}$-cycloalkyl and heterocyclyl rings may optionally contain one or more unsaturated bonds situated in such a way, however, that an aromatic $\pi$-electron system does not arise.

Illustrative examples of "$C_{3-6}$-cycloalkyl" are the carbocycles cyclopropane, cyclobutane, cyclopentane, cyclopentene, cyclopentadiene, cyclohexane, cyclohexene, 1,3-cyclohexadiene and 1,4-cyclohexadiene.

Illustrative examples of "heterocyclyls" are the nitrogen-containing heterocycles 2-pyrrolinyl, 3-pyrrolinyl, pyrrolidinyl, 2-imidazolinyl, imidazolidinyl, 2-pyrazolinyl, 3-pyrazolinyl, pyrazolidinyl, piperidinyl, morpholinyl, thiomorpholinyl and piperazinyl. Binding to the heterocycle may be at the position of the heteroatom or via a carbon atom of the heterocycle.

In the present context, the term "$C_{2-6}$-alkenyl" is intended to mean a linear or branched hydrocarbon group having from two to six carbon atoms and containing one or more double bonds. Illustrative examples of $C_{2-6}$-alkenyl groups include allyl, homo-allyl, vinyl, crotyl, butenyl, pentenyl and hexenyl. Illustrative examples of $C_{2-6}$-alkenyl groups with more than one double bond include butadienyl, pentadienyl and hexadienyl. The position of the double bond(s) may be at any position along the carbon chain.

In the present context the term "$C_{2-6}$-alkynyl" is intended to mean a linear or branched hydrocarbon group

containing from two to six carbon atoms and containing one or more triple bonds. Illustrative examples of $C_{2-6}$-alkynyl groups include acetylene, propynyl, butynyl, pentynyl and hexynyl. The position of the triple bond(s) may be at any position along the carbon chain. More than one bond may be unsaturated so that the "$C_{2-6}$-alkynyl" is a di-yne or enedi-yne as is known to the person skilled in the art.

When used herein the term "$C_{1-6}$-alkoxy" is intended to mean $C_{1-6}$-alkyl-oxy, such as methoxy, ethoxy, n-propoxy, isopropoxy, n-butoxy, isobutoxy, sec-butoxy, *tert*-butoxy, n-pentoxy, isopentoxy, neopentoxy and n-hexoxy.

The term "halogen" includes fluorine, chlorine, bromine and iodine.

In the present context the term "aryl" is intended to mean a carbocyclic aromatic ring or ring system. Moreover, the term "aryl" includes fused ring systems wherein at least two aryl rings, or at least one aryl and at least one $C_{3-6}$-cycloalkyl, or at least one aryl and at least one heterocyclyl, share at least one chemical bond. Illustrative examples of "aryl" rings include phenyl, naphthalenyl, phenanthrenyl, anthracenyl, acenaphthylenyl, tetralinyl, fluorenyl, indenyl, indolyl, coumaranyl, coumarinyl, chromanyl, isochromanyl, and azulenyl.

In the present context, the term "heteroaryl" is intended to mean an aryl group where one or more carbon atoms in an aromatic ring have been replaced with one or more heteroatoms selected from the group consisting of nitrogen, sulphur, phosphorous and oxygen. Furthermore, in the present context, the term "heteroaryl" comprises fused ring systems wherein at least one aryl ring and at least one heteroaryl ring, at least two heteroaryls, at least one heteroaryl and at least one heterocyclyl, or at least one heteroaryl and at least one $C_{3-6}$-cycloalkyl share at least one chemical bond.

Illustrative examples of a heteroaryl include furanyl, thienyl, pyrrolyl, phenoxazonyl, oxazolyl, thiazolyl, isothiazolyl, imidazolyl, pyrazolyl, isoxazolyl, imidazolyl isothiazolyl, oxadiazolyl, furazanyl, triazolyl, thiadiazolyl, piperidinyl, pyridinyl, pyridazinyl, pyrimidinyl, pyrazinyl, pyrazolyl and triazinyl, isoindolyl, indolinyl, benzofuranyl, benzothiophenyl, benzopyrazolyl,indazolyl, benzimidazolyl, benzthiazolyl, purinyl, quinolizinyl, quinolinyl, isoquinolinyl, cinnolinyl, phthalazinyl, quinazolinyl, quinoxalinyl, naphthyridinyl, pteridinylthienofuranyl, carbazolyl, acridinyl, phenazinyl, phenothiazinyl, phenoxazinyl and thianthrenyl.

In the present context the term "optionally substituted" is intended to mean that the group in question may be substituted one or several times, such as 1 to 5 times, preferably 1 to 3 times, most preferably 1 to 2 times, with one or more groups selected from the group consisting of $C_{1-6}$-alkyl, $C_{1-6}$-alkoxy, oxo (which may be represented in the tautomeric enol form), carboxyl, amino, hydroxy (which when present in an enol system may be represented in the tautomeric keto form), nitro, sulphono, sulphanyl, $C_{1-6}$-carboxyl, $C_{1-6}$-alkoxycarbonyl, $C_{1-6}$-alkylcarbonyl, formyl, aryl, aryloxy, aryloxycarbonyl, arylcarbonyl, heteroaryl, amino, mono- and di($C_{1-6}$-alkyl)amino, carbamoyl, mono- and di($C_{1-6}$-alkyl)aminocarbonyl, amino-$C_{1-6}$-alkyl-aminocarbonyl, mono- and di($C_{1-6}$-alkyl)amino-$C_{1-6}$-alkyl-aminocarbonyl, $C_{1-6}$-alkylcarbonylamino, cyano, guanidino, carbamido, $C_{1-6}$-alkanoyloxy, $C_{1-6}$-alkylsulphonyloxy, dihalogen-$C_{1-6}$-alkyl, trihalogen-$C_{1-6}$-alkyl and halogen, where aryl and heteroaryl substituents may themselves be substituted 1-3 times with $C_{1-6}$-alkyl, $C_{1-6}$-alkoxy, nitro, cyano, hydroxy, amino or halogen. In general, the above substituents may be susceptible to further optional substitution.

The term "infectious agent" is intended to mean pathogenic microorganisms, such as bacteria, viruses, fungi and intra- or extra-cellular parasites.

Analogously, the term "infectious disease" is used about a disease caused by an infectious agent.

In the present context, the term "anti-infective agent" covers compounds, such as commercially available antibiotics, that are capable of killing, inhibiting or otherwise slowing the growth of the infectious agent.

Specific examples of antibiotics commonly used for treating bacterial and fungal infections include, but is not limited to, aminoglycosides, such as amikacin, gentamicin, kanamycin, neomycin, netilmicin, streptomycin and tobramycin; cabecephems, such as loracarbef; carbapenems, such as ertapenem, imipenem/cilastatin and meropenem; cephalosporins, such as cefadroxil, cefazolin, cephalexin, cefaclor, cefamandole, cephalexin, cefoxitin, cefprozil, cefuroxime, cefixime, cefdinir, cefditoren, cefoperazone, cefotaxime, cefpodoxime, ceftazidime, ceftibuten, ceftizoxime, ceftriaxone and cefepime; macrolides, such as azithromycin, clarithromycin, dirithromycin, erythromycin and troleandomycin; monobactam; penicillins, such as amoxicillin, ampicillin, carbenicillin, cloxacillin, dicloxacillin, nafcillin, oxacillin, penicillin G, penicillin V, piperacillin and ticarcillin; polypeptides, such as bacitracin, colistin and polymyxin B; quinolones, such as ciprofloxacin, enoxacin, gatifloxacin, levofloxacin, lomefloxacin, moxifloxacin, norfloxacin, ofloxacin and trovafloxacin; sulfonamides, such as mafenide, sulfacetamide, sulfamethizole, sulfasalazine, sulfisoxazole and trimethoprim-sulfamethoxazole;

tetracyclines, such as demeclocycline, doxycycline, minocycline, oxytetracycline and tetracycline;

Specific examples of anti-viral compounds commonly used for treating viral infections include, but is not limited to, acyclovir, amantadine, cidofovir famciclovir, fomivirsen, foscarnet, ganciclovir, interferon alpha, oseltamivir, penciclovir, ribavirin, rimantadine, trifluridine, valacyclovir, valganciclovir, vidarabine and zanamivir.

Specific examples of anti-fungal compounds commonly used for treating severe fungal infections include, but is not limited to, amphotericin B, caspofungin, fluconazole, flucytosine, itraconazole, ketoconazole and voriconazole.

In the present context, an infectious agent is said to be "resistant" or "drug resistant" if the infectious agent has undergone a change which reduces or eliminates the effectiveness of an anti-infective agent which is normally used to

cure infections caused by the infectious agent. Analogously, the term "drug resistance" means a circumstance when a disease, e.g. an infectious disease, does not respond to a therapeutic agent, such as an anti-infective agent. Drug resistance can be intrinsic, which means that the disease has never been responsive to the therapeutic agent, or acquired, which means that the disease ceases responding to the therapeutic agent to which the disease had previously been responsive.

In the present context, an infectious agent is said to be "multidrug resistant" if the infectious agent has undergone a change which reduces or eliminates the effectiveness of two or more anti-infective agents which are normally used to cure infections caused by the infectious agent. Analogously, "multidrug resistance" is a type of drug resistance wherein a disease, e.g. an infectious disease, is resistant to a variety of drugs, such as a variety of anti-infective agents.

The term "clinically relevant amount" is intended to mean that the chemosensitising compound is administered to a patient in an amount, which, on the one hand, is capable of reducing the symptoms of the infectious disease or curing the infectious disease for which the patient is treated, but, on the other hand, is not toxic to the patient and does not lead to unacceptable side effects. As indicated above, many, if not all, of the chemosensitising compounds described herein are known to cause severe side effects in patients when administered in too high concentrations, i.e. in amounts which are not "clinically relevant".

In the present context, the term "naturally occurring" when used in connection with the term "infectious agent", i.e. in connection with pathogenic microorganisms, means that the infectious agent giving rise to the infectious disease is a microorganism that can be found in nature, including in human beings. It will be understood that infectious agents, such as gen-manipulated laboratory strains, or infectious agents which by other means have been changed and/or manipulated by human intervention, are not considered to be covered by the term "naturally occurring".

The term "serum" is used in its normal meaning, i.e. as blood plasma without fibrinogen and other clotting factors.

In order to combat or prevent the infectious disease, the chemosensitising compounds disclosed herein should be administered together with, or in combination with, an anti-infective agent. When used in this context the terms "together with" and "in combination with" should not be interpreted narrowly in the sense that the chemosensitising compound and the anti-infective agent should necessarily be administered simultaneously and/or form part of the same pharmaceutical composition, although this is one embodiment of the present invention. Thus, it should be understood that the terms "together with" and "in combination with" mean that the dosage (including the dosage form) and the administration frequency of each compound, i.e. the chemosensitising compound and the anti-infective agent, may be controlled individually. For example, one compound may be administered orally three times per day during the treatment period, while the other compound may be administered intravenously once per day during the treatment period. Likewise, one compound may be administered every day in the treatment period and the other compound may be administered only once or a few days in the treatment period. As explained above, the chemosensitising compound and the anti-infective compound may be administered simultaneously and they may be comprised in the same pharmaceutical composition. Accordingly, the terms "together with" and "in combination with" mean that the chemosensitising compound is administered to the patient at least once during the treatment period and that the anti-infective agent is also administered to the patient at least once during the treatment period.

Herein, the term "steady state serum concentration" (of a chemosensitising compound) is defined as those values that recur with each dose and represent a state of equilibrium between the amount of chemosensitising compound administered and the amount being eliminated in a given time interval.

In the present context, the term "treatment" refers to the administration of a drug to a subject and includes i) preventing an infectious disease (i.e. causing the clinical symptoms of the infectious disease not to develop), *ii)* inhibiting an infectious disease (i.e. arresting the development of the clinical symptoms of the infectious disease) and *iii)* relieving the disease (i.e. causing regression of the clinical symptoms of the infectious disease) as well as combinations thereof.

The terms "prophylaxis" or "prophylactic treatment" refers to the treatment of a subject who is not yet infected, but who may be susceptible to, or at risk of getting an infection.

The term "subject", as used herein, means a living vertebrate animal, e.g., a mammal, such as a human being.

"Pharmaceutically acceptable" means suitable for use in a mammal, in particular suitable for use in a human being.

When used herein, the term "chemosensitising compound" is intended to mean an agent which reverses a microorganism's or cell's resistance to a given anti-infective agent. Thus, a "chemosensitising compound", as used herein, sensitises resistant or multidrug resistant microorganisms or cells to the action of anti-infective agents.

**Chemosensitising compounds**

Concerning the general formula (I) above, the substituents $R_1$, $R_2$, $R_3$, $R_4$, $R_5$, $R_6$, $R_7$, $R_8$ and $R_9$ are each individually selected from the group consisting of hydrogen, halogen, hydroxy, amino, nitro, optionally substituted $C_{1-6}$-alkyl, optionally substituted $C_{2-6}$-alkenyl, optionally substituted $C_{2-6}$-alkynyl and optionally substituted $C_{1-6}$-alkoxy, optionally substituted $C_{2-6}$-alkenyloxy, carboxy, optionally substituted $C_{1-6}$-alkoxycarbonyl, optionally substituted

$C_{1-6}$-alkylcarbonyl, fomyl, optionally substituted $C_{1-6}$-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, mono- and di($C_{1-6}$-alkyl)amino, carbamoyl, mono- and di($C_{1-6}$-alkyl)aminocarbonyl, amino-$C_{1-6}$-alkyl-aminocarbonyl, mono- and di($C_{1-6}$-alkyl)amino-$C_{1-6}$-alkyl-aminocarbonyl, $C_{1-6}$-alkylcarbonylamino, amino-$C_{1-6}$-alkylcarbonylamino, mono- and di($C_{1-6}$-alkyl)amino-$C_{1-6}$-alkyl-carbonylamino, amino-$C_{1-6}$-alkyl-amino, mono- and di($C_{1-6}$-alkyl)amino-$C_{1-6}$-alkyl-amino, cyano, guanidino, carbamido, $C_{1-6}$-alkanoyloxy, $C_{1-6}$-alkylsulphonyl, $C_{1-6}$-alkylsulphinyl, $C_{1-6}$-alkylsulphonyloxy, aminosulfonyl, mono- and di($C_{1-6}$-alkyl)aminosulfonyl, and optionally substituted $C_{1-6}$-alkylthio.

In a preferred embodiment of the invention, the $R_2$ substituent is an electron-withdrawing group, such as halogen, nitro or halogen-substituted $C_{1-6}$-alkyl. More preferably, $R_2$ is selected from the group consisting of F, Cl, Br, I, $CH_2Y$, $CHY_2$ and $CY_3$ (wherein Y represents a halogen atom), such as $CH_2Cl$, $CH_2F$, $CHCl_2$, $CHF_2$, $CCl_3$ or $CF_3$, in particular $CCl_3$ or $CF_3$. Most preferably, $R_2$ is Cl or $CF_3$.

The substituents $R_1$, $R_3$, $R_4$, $R_5$, $R_6$, $R_7$, $R_8$ and $R_9$ are preferably each individually selected from the group consisting of hydrogen, optionally substituted $C_{1-6}$-alkyl and optionally substituted $C_{1-6}$-alkoxy. More preferably, all of $R_1$, $R_3$, $R_4$, $R_5$, $R_6$, $R_7$, $R_8$ and $R_9$ are hydrogen.

Accordingly, in a highly preferred embodiment of the invention, $R_2$ is Cl or $CF_3$ and each of $R_1$, $R_3$, $R_4$, $R_5$, $R_6$, $R_7$, $R_8$ and $R_9$ are hydrogen.

As mentioned above, V is selected from the group consisting of S, $SO_2$, SO, O and NH, such as S or SO. In a highly preferred embodiment of the invention, V is S.

As will be understood, in case W is N-$(CHX)_n$-N($R_{10}$)($R_{11}$) and V is S, the chemosensitising compound of the general formula (I) becomes a phenothiazine of the

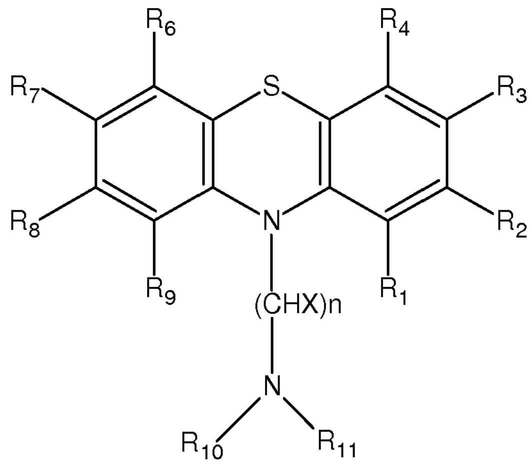

(II)

general formula (II)

wherein n is an integer in the range of from 2 to 6, such as 2, 3, 4, 5 or 6, and each X is individually selected from the group consisting of hydrogen, halogen, hydroxy, amino, nitro, optionally substituted $C_{1-6}$-alkyl and optionally substituted $C_{1-6}$-alkoxy. $R_{10}$ and $R_{11}$ are each independently selected from the group consisting of hydrogen, optionally substituted $C_{1-6}$-alkyl, optionally substituted $C_{2-6}$-alkenyl, optionally substituted $C_{2-6}$-alkynyl, optionally substituted $C_{1-6}$-alkoxycarbonyl, optionally substituted $C_{1-6}$-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di($C_{1-6}$-alkyl)aminocarbonyl; or $R_{10}$ and $R_{11}$ together with the nitrogen atom to which they are attached form an optionally substituted nitrogen-containing heteroaryl or optionally substituted heterocyclyl.

In a preferred embodiment of the invention n is 2 or 3 and X is hydrogen or $CH_3$. Thus in a preferred embodiment of the invention, W is N-$(CH_2)_3$-N($R_{10}$)($R_{11}$) or N-$CH_2$-CH($CH_3$)-N($R_{10}$)($R_{11}$). In particular, the structure where W is N-$(CH_2)_3$-N($R_{10}$)($R_{11}$) is preferred.

In one interesting embodiment of the invention, $R_{10}$ and $R_{11}$ are each individually selected from the group consisting of hydrogen and optionally substituted $C_{1-6}$-alkyl. According to this embodiment, it is preferred that both of $R_{10}$ and $R_{11}$ are optionally substituted $C_{1-6}$-alkyl. Most preferably both of $R_{10}$ and $R_{11}$ are $CH_3$.

In another interesting embodiment of the invention, $R_{10}$ and $R_{11}$, together with the nitrogen atom to which they are attached, form an optionally substituted heterocyclyl, such as optionally substituted 2-pyrrolinyl, optionally substituted 3-pyrrolinyl, optionally substituted pyrrolidinyl, optionally substituted 2-imidazolinyl, optionally substituted imidazolidinyl, optionally substituted 2-pyrazolinyl, optionally substituted 3-pyrazolinyl, optionally substituted pyrazolidinyl, optionally substituted piperidinyl, optionally substituted morpholinyl, optionally substituted thiomorpholinyl or optionally substituted piperazinyl, According to this embodiment, it is preferred that $R_{10}$ and $R_{11}$, together with the nitrogen atom to which they are attached, form an optionally substituted piperidinyl or an optionally substituted piperazinyl, in particular an optionally substituted piperazinyl. The piperazinyl ring may be unsubstituted, but is preferably substituted with an optionally substituted $C_{1-6}$ alkyl group, in particular in the *para* position, i.e. an optionally substituted $C_{1-6}$ alkyl group is covalently attached to the second nitrogen atom of the piperazinyl ring. In a highly preferred embodiment of the invention, the optionally substituted $C_{1-6}$-alkyl is selected from the group consisting of $-CH_3$, $-CH_2OH$, $-CH_2-CH_3$ and $-CH_2-CH_2OH$, such as- $CH_3$ or $-CH_2-CH_2OH$, in particular $-CH_2-CH_2OH$.

Specific examples of the above-mentioned phenothiazines include promazine, 1-chlorpromazine, chlorpromazine, 7-hydroxychlorpromazine, 7,8-dihydroxychlorpromazine, thiomethlypromazine, trifluorpromazine, desmethylclorpromazine, perphenazine, prochlorperazine and 2-chloro-1 0-(2-dimethylaminoethyl)phenothiazine.

As will also be understood, in case W is C=CH-$(CHX)_m$-N($R_{10}$)($R_{11}$) and V is S, the chemosensitising compound of the general formula (I) becomes a thioxanthene of the general formula (III)

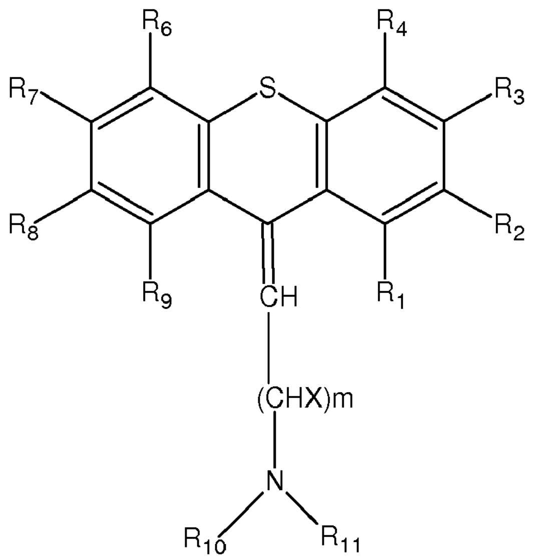

(III)

A thioxanthene of the general formula (III) gives rise to *cis* and *trans* isomerism. In the present context, compounds of the general formula (IIIa) are said to be in the *cis* configuration, whereas compounds of the general formula (IIIb) are said to be in the *trans* configuration:

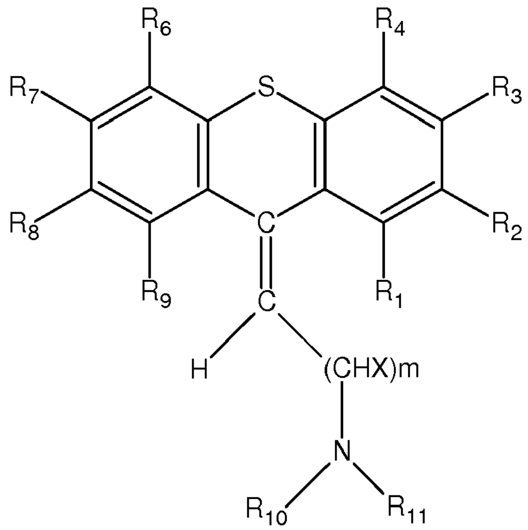

(IIIa)

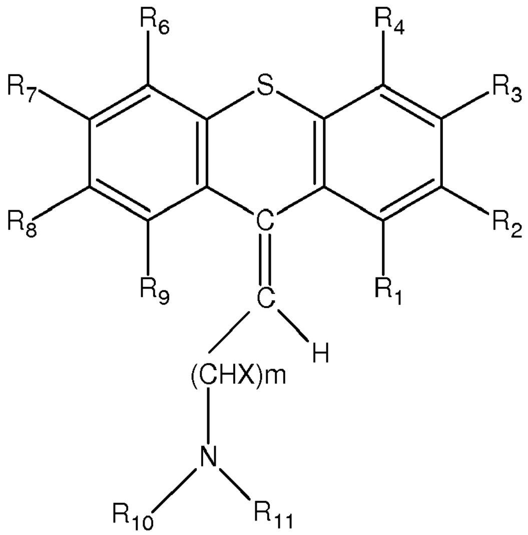

(IIIb)

wherein m is an integer in the range of from 1 to 5, such as 1, 2, 3, 4, or 5, and each X is individually selected from the group consisting of hydrogen, halogen, hydroxy, amino, nitro, optionally substituted $C_{1-6}$-alkyl and optionally substituted $C_{1-6}$-alkoxy. $R_{10}$ and $R_{11}$ are each independently selected from the group consisting of hydrogen, optionally substituted $C_{1-6}$-alkyl, optionally substituted $C_{2-6}$-alkenyl, optionally substituted $C_{2-6}$-alkynyl, optionally substituted $C_{1-6}$-alkoxycarbonyl, optionally substituted $C_{1-6}$-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di($C_{1-6}$-alkyl)aminocarbonyl; or $R_{10}$ and $R_{11}$ together with the nitrogen atom to which they are attached form an optionally substituted nitrogen-containing heteroaryl or optionally substituted heterocyclyl.

It is generally preferred that the compounds of the general formula (III) have the *trans* configuration, i.e. the structure shown in the general formula (IIIb).

In a preferred embodiment, X is hydrogen and m is 2 or 3, in particular 3. Thus, in a preferred embodiment of the invention, W has the structure $C=CH-(CH_2)_2-N(R_{10})(R_{11})$.

In one interesting embodiment of the invention, $R_{10}$ and $R_{11}$ are each individually selected from the group consisting of hydrogen and optionally substituted $C_{1-6}$-alkyl. According to this embodiment, it is preferred that both of $R_{10}$ and $R_{11}$ are optionally substituted $C_{1-6}$-alkyl. Most preferably both of $R_{10}$ and $R_{11}$ are $CH_3$.

In another interesting embodiment of the invention, $R_{10}$ and $R_{11}$, together with the nitrogen atom to which they are attached, form an optionally substituted heterocyclyl, such as optionally substituted 2-pyrrolinyl, optionally substituted 3-pyrrolinyl, optionally substituted pyrrolidinyl, optionally substituted 2-imidazolinyl, optionally substituted imidazolidinyl, optionally substituted 2-pyrazolinyl, optionally substituted 3-pyrazolinyl, optionally substituted pyrazolidinyl, optionally substituted piperidinyl, optionally substituted morpholinyl, optionally substituted thiomorpholinyl or optionally substituted piperazinyl, According to this embodiment, it is preferred that $R_{10}$ and $R_{11}$, together with the nitrogen atom to which they are attached, form an optionally substituted piperidinyl or an optionally substituted piperazinyl, in particular an optionally substituted piperazinyl. The piperazinyl ring may be unsubstituted, but is preferably substituted with an optionally substituted $C_{1-6}$ alkyl group, in particular in the *para* position, i.e. an optionally substituted $C_{1-6}$ alkyl group is covalently attached to the second nitrogen atom of the piperazinyl ring. In a highly preferred embodiment of the invention, the optionally substituted $C_{1-6}$-alkyl is selected from the group consisting of $-CH_3$, $-CH_2OH$, $-CH_2-CH_3$ and ,$CH_2-CH_2OH$, such as $-CH_3$ or $-CH_2-CH_2OH$, in particular $-CH_2-CH_2OH$.

Specific examples of the above-mentioned phenothiazines include trans-flupenthixol, cis-flupenthixol, *trans*-clopenthixol and cis- clopenthixol. Particularly preferred chemosensitising compounds for the use according to the invention are trans-flupenthixol and *trans*-clopenthixol. Most preferred is *trans*-clopenthixol.

As is evident from the formulae shown herein and the definitions associated therewith, certain of the chemosensitising compounds described herein are chiral. Moreover, the presence of certain unsaturated or cyclic fragments or multiple stereogenic atoms provides for the existence of diastereomeric forms of some of the chemosensitising compounds. The invention is intended to include all stereoisomers, including optical isomers, and mixtures thereof, as well as pure, partially enriched, or, where relevant, racemic forms. In particular, many of the chemosensitising compounds described herein may be in the form of *E*- or *Z*-stereoisomers, or mixtures of such isomers.

It should furthermore be understood that the chemosensitising compounds described herein include possible salts thereof, of which pharmaceutically acceptable salts are of course especially relevant for the therapeutic applications. Salts include acid addition salts and basic salts. Examples of acid addition salts are hydrochloride salts, fumarate, oxalate, etc. Examples of basic salts are salts where the (remaining) counter ion is selected from alkali metals, such as sodium and potassium, alkaline earth metals, such as calcium salts, potassium salts, and ammonium ions ($^+N(R')_4$, where the R's independently designate optionally substituted $C_{1-6}$-alkyl, optionally substituted $C_{2-6}$-alkenyl, optionally substituted aryl, or optionally substituted heteroaryl). Pharmaceutically acceptable salts are, e.g., those described in Remington's - The Science and Practice of Pharmacy, 20th Ed. Alfonso R.Gennaro (Ed.), Lippincott, Williams & Wilkins; ISBN: 0683306472, 2000, and in Encyclopedia of Pharmaceutical Technology.

The effect of the chemosensitising compounds for reversing drug resistance or multiple drug resistance may be assayed as described herein and the efficiency of the chemosensitising compound in combination with selected anti-infective agents against selected microorganisms may be expresses as the MIC value, DR ratio and/or the FIC index.

The Minimal Inhibitory Concentration, (MIC) is defined as the lowest inhibitory concentration showing no visible growth according to the NCCLS Guidelines.

The Drug Resistance (DR) ratio is defined as the ratio between the MIC value for anti-infective agent alone divided by the MIC for the anti-infective agent in the presence of the chemosensitising compound. This ratio represents the increase in apparent potency of the anti-infective agent caused by the chemosensitising compound, and may be expressed as

$$\text{DR ratio} = (\text{MIC}_{\text{anti-infective agent}})/(\text{MIC}_{\text{anti-infective agent + chemosensitising compound}})$$

The Fractional Inhibitory Concentration (FIC) index may be calculated for each anti-infective agent alone and in combination with chemosensitising according to the following formulae:

$$\text{FIC} = \text{FIC}_{\text{chemosensitising compound}} + \text{FIC}_{\text{anti-infective agent}}$$

where:

$$\text{FIC}_{\text{chemosensitising compound}} = (\text{MIC}_{\text{chemosensitising compound + anti-infective agent}})/(\text{MIC}_{\text{chemosensitising compound}})$$

$$FIC_{\text{anti-infective agent}} = (MIC_{\text{anti-infective agent + chemosensitising compound}}/(MIC_{\text{anti-infective agent}})$$

The synergistic effects of the chemosensitising compounds described herein, i.e. their ability to reverse drug resistance or multiple drug resistance in a microorganism, may be assessed by any of the methods available to those skilled in the art, including the *in vitro* assays described in the examples herein. In a preferred embodiment of the invention, the chemosensitising compound, the anti-infective agent and the infectious agent (and hence the infectious disease to be treated) exhibit a FIC index of at the most 0.5 when determined as described in the examples herein. More preferably, the FIC index is at the most 0.4, such as at the most 0.3, e.g. at the most 0.2. Even more preferably, the FIC index is at the most 0.1, such as at the most 0.075, at the most 0.05 or even at the most 0.025.

As described previously, the chemosensitising compounds described herein typically have a high MIC value. For chemosensitising compounds, which are effective inhibitors, this means that the ratio $(MIC_{\text{chemosensitising compound + anti-infective agent}})/(MIC_{\text{chemosensitising compound}})$ becomes close to zero, which, in turn, means that $FIC_{\text{chemosensitising compound}} \approx 0$. This also means that $FIC \approx FIC_{\text{anti-infective agent}} = (MIC_{\text{anti-infective agent + chemosensitising compound}}/(MIC_{\text{anti-infective agent}}) \approx 1/DR$.

Accordingly, in another preferred embodiment of the invention, the chemosensitising compound, the anti-infective agent and the infectious agent (and hence the infectious disease to be treated) exhibit a DR ratio of at least 2 when determined as described in the examples herein. More preferably, the DR ratio is at least 5, such as at least 10, e.g. at least 20. Even more preferably, the MIC value is at least 30, such as at least 50, at least 75 or even at least 100.

**Therapy, pharmaceutical compositions and dosages**

As explained above, the chemosensitising compounds described herein are useful for treatment of infectious diseases in combination with an anti-infective agent. Thus, the chemosensitising compounds described herein may be used for the manufacture of a medicament for the treatment of an infectious disease in combination with an anti-infective agent.

In addition, the chemosensitising compounds described herein are useful for prophylactic treatment of infectious diseases in combination with an anti-infective agent. This may be particularly relevant in situations where a person has a high risk of getting infections, such as immunosuppressed patients or patients undergoing surgery. Thus, the chemosensitising compounds described herein may also be used for the manufacture of a medicament for the prophylactic treatment of an infectious disease in combination with an anti-infective agent.

Also, the chemosensitising compounds described herein may be used, in combination with an anti-infective agent, for the manufacture of a medicament for the treatment or prophylaxis of infectious diseases.

In a further aspect, the present invention is directed to the chemosensitising compounds described herein for use as medicaments in combination with an anti-infective agent, or to the chemosensitising compounds described herein, in combination with an anti-infective agent, for use as medicaments.

In another, but related, aspect, the chemosensitising compounds described herein are useful for decreasing resistance, in particular multidrug resistance, of an infectious agent. Thus, the chemosensitising compounds described herein may be used for the manufacture of a medicament for decreasing resistance of an infectious agent against an anti-infective agent.

In still another, but also related aspect, the chemosensitising compounds described herein are useful for sensitising susceptible, resistant or multidrug resistant cells, preferably resistant and multidrug resistant cells, more preferably multidrug resistant cells, to an anti-infective agent. Thus, the chemosensitising compounds described herein may be used for the manufacture of a medicament for sensitising susceptible, resistant or multidrug resistant cells, preferably resistant and multidrug resistant cells, more preferably multidrug resistant cells, to an anti-infective agent.

A further aspect of the present invention relates to a method for treating or preventing an infectious disease in a subject, said method comprising administering to said subject a chemosensitising compound as described herein in combination with an anti-infective agent.

A still further aspect of the present invention relates to a method for decreasing resistance, in particular multidrug resistance, of an infectious agent.

An even further aspect of the present invention relates for a method for sensitising susceptible, resistant or multidrug resistant microorganisms or cells, preferably resistant and multidrug resistant microorganisms or cells, more preferably multidrug resistant microorganisms or cells, to an anti-infective agent.

Still another aspect of the present invention relates to a method for prevention of development of resistance or multidrug resistance in an infectious agent, said method comprising administering to a subject a chemosensitising compound as described herein.

Therapy

As will be understood from the disclosure herein, the infectious disease to be treated is one that is normally treated with an anti-infective agent. The infectious disease is normally caused by an infectious agent, such as a bacterium, a virus, a fungi or an intra- or extra-cellular parasite, in particular a bacterium. The infectious agent is typically naturally-occurring, i.e. a naturally-occurring bacterium, a naturally occurring virus, a naturally occurring fungi or a naturally occurring intra- or extra-cellular parasite, in particular a naturally-occurring bacterium.
More particularly, the infectious agent may be Gram negative or Gram positive bacteria.
Specific examples include Gram negative bacteria of a genus selected from the group consisting of Escherichia, Proteus, Salmonella, Klebsiella, Providencia, Enterobacter, Burkholderia, Pseudomonas, Acinetobacter, Aeromonas, Haemophilus, Yersinia, Neisseria, Erwinia, Rhodopseudomonas and Burkholderia.
Specific examples of Gram positive bacteria include bacteria from a genus selected from the group consisting of Lactobacillus, Azorhizobium, Streptococcus, Pediococcus, Photobacterium, Bacillus, Enterococcus, Staphylococcus, Clostridium, Butyrivibrio, Sphingomonas, Rhodococcus and Streptomyces.
In other embodiments, the infectious agent is, e.g., from a genus selected from the group consisting of Methanobacierium, Sulfolobus, Archaeoglobu, Rhodobacter and Sinorhizobium.
In still other embodiments, the infectious agent is fungi, such as from the genus Mucor or Candida, e.g., Mucor racemosus or Candida albicans; from genus Crytococcus e.g., Cr. Neoformans; or from Genus Aspergillus, e.g., A. fumingatus.
In yet other embodiments, the infectious agent is protozoa, such as a malaria or cryptosporidium parasite.
Toxicity and therapeutic efficacy of the chemosensitising compounds described herein can be determined by standard pharmaceutical procedures in cell cultures or experimental animals, e.g., by determining the $LD_{50}$ (the dose lethal for 50% of the population) and the $ED_{50}$ (the dose therapeutically effective in 50% of the population). The dose ratio between toxic and therapeutic effects is the therapeutic index and it can be expressed as the ratio between $LD_{50}$ and $ED_{50}$ ($LD_{50}/ED_{50}$). Chemosensitising compounds which exhibit large therapeutic indices are preferred. The data obtained from these cell culture assays or animal studies can be used in formulating a range of dosage for use in human subjects. The dosage of such chemosensitising compounds lies preferably within a range of circulating concentrations that include the $ED_{50}$ with little or no toxicity. The dosage may vary within this range depending upon the dosage form employed and the route of administration utilised.

Pharmaceutical compositions

The chemosensitising compounds described herein are typically formulated in a pharmaceutical composition prior to use as a drug substance.
Accordingly, in a further aspect the present invention relates to a pharmaceutical composition comprising a chemosensitising compound as described herein and at least one pharmaceutically acceptable carrier or exipient.
The pharmaceutical composition may contain a single or two or more chemosensitising compound as described herein. In an interesting embodiment of the invention the pharmaceutical composition comprises, in addition to a chemosensitising compound, an anti-infective agent.
The administration route of the chemosensitising compounds described herein may be any suitable route that leads to a concentration in the blood or tissue corresponding to a clinically relevant concentration. Thus, e.g., the following administration routes may be applicable although the invention is not limited thereto: the oral route, the parenteral route, the cutaneous route, the nasal route, the rectal route, the vaginal route and the ocular route. It should be clear to a person skilled in the art that the administration route is dependant on the particular chemosensitising compound in question, particularly, the choice of administration route depends on the physico-chemical properties of the chemosensitising compound together with the age and weight of the patient and on the particular disease or condition and the severity of the same. In general, however, the oral and the parental routes are preferred.
The chemosensitising compounds described herein may be contained in any appropriate amount in the pharmaceutical composition, and are generally contained in an amount of about 0.1-95% by weight of the total weight of the composition. The composition may be presented in a dosage form, such as a unit dosage form, which is suitable for the oral, parenteral, rectal, cutaneous, nasal, vaginal and/or ocular administration route. Thus, the composition may be in form of, e.g., tablets, capsules, pills, powders, granulates, suspensions, emulsions, solutions, gels including hydrogels, pastes, ointments, creams, plasters, drenches, delivery devices, suppositories, enemas, injectables, implants, sprays, aerosols and in other suitable form.
The pharmaceutical compositions may be formulated according to conventional pharmaceutical practice, see, e.g., "Remington's Pharmaceutical Sciences" and "Encyclopedia of Pharmaceutical Technology", edited by Swarbrick, J. & J. C. Boylan, Marcel Dekker, Inc., New York, 1988. Typically, the chemosensitising compounds described herein are formulated with (at least) a pharmaceutically acceptable carrier or exipient. Pharmaceutically acceptable carriers or

exipients are those known by the person skilled in the art.

Pharmaceutical compositions for oral use include tablets which contain a chemosensitising compound as described herein, optionally in combination with at least one anti-infective agent, in admixture with non-toxic pharmaceutically acceptable excipients. These excipients may be, for example,
inert diluents or fillers, such as sucrose, sorbitol, sugar, mannitol, microcrystalline cellulose, starches including potato starch, calcium carbonate, sodium chloride, lactose, calcium phosphate, calcium sulfate or sodium phosphate;
granulating and disintegrating agents, for example, cellulose derivatives including microcrystalline cellulose, starches including potato starch, croscarmellose sodium, alginates or alginic acid;
binding agents, for example, sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone or polyethylene glycol; and
lubricating agents, including glidants and antiadhesives, for example, magnesium stearate, zinc stearate, stearic acid, silicas, hydrogenated vegetable oils or talc.

Other pharmaceutically acceptable excipients can be colorants, flavouring agents, plasticisers, humectants, buffering agents, etc.

The tablets may be uncoated or they may be coated by known techniques, optionally to delay disintegration and absorption in the gastrointestinal tract and thereby providing a sustained action over a longer period. The coating may be adapted to release the chemosensitising compound in a predetermined pattern, e.g., in order to achieve a controlled release formulation (see below) or it may be adapted not to release the active drug substance until after passage of the stomach (enteric coating). The coating may be a sugar coating, a film coating (e.g. based on hydroxypropyl methylcellulose, methylcellulose, methyl hydroxyethylcellulose, hydroxypropylcellulose, carboxymethylcellulose, acrylate copolymers (Eudragit E®), polyethylene glycols and/or polyvinylpyrrolidone) or an enteric coating (e.g. based on methacrylic acid copolymer (Eudragit® L and S), cellulose acetate phthalate, hydroxypropyl methylcellulose phthalate, hydroxypropyl methylcellulose acetate succinate, polyvinyl acetate phthalate, shellac and/or ethylcellulose).

Furthermore, a time delay material such as, e.g., glyceryl monostearate or glyceryl distearate may be employed.

In addition, the solid tablet compositions as mentioned above may be provided with a coating adapted to protect the composition from unwanted chemical changes, e.g. chemical degradation, prior to the release of the chemosensitising compound.

The coating may be applied on the solid dosage form in a similar manner as that described in "Aqueous film coating" by James A. Seitz in "Encyclopedia of Pharmaceutical Technology", Vol 1, pp.337-349 edited by Swarbrick, J. & J. C. Boylan, Marcel Dekker, Inc., New York, 1988.

Formulations for oral use may also be presented as chewing tablets, or as hard gelatin capsules wherein the active ingredient is mixed with an inert solid diluent, for example, potato starch, lactose, microcrystalline cellulose, calcium carbonate, calcium phosphate or kaolin, or as soft gelatin capsules wherein the active ingredient is mixed with water or an oil medium, for example, peanut oil, liquid paraffin, or olive oil.

Powders and granulates may be prepared using the ingredients mentioned above under tablets and capsules in a conventional manner using, e.g., a mixer, a fluid bed apparatus or a spray drying equipment.

Controlled release compositions for oral use may, e.g., be constructed to release the active drug substance by controlling the dissolution and/or the diffusion of the active drug substance.

Dissolution or diffusion controlled release can be achieved by appropriate coating of a tablet, capsule, pellet or granulate formulation of the chemosensitising compound, or by incorporating the chemosensitising compound in question in, e.g., an appropriate matrix.

A controlled release coating may comprise one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3-butylene glycol, ethylene glycol methacrylate and/or polyethylene glycols.

In a controlled release matrix formulation of the chemosensitising compound, the matrix material may comprise, e.g., hydrated metylcellulose, carnauba wax and stearyl alcohol, carbopol 934, silicone, glyceryl tristearate, methyl acrylate-methyl methacrylate, polyvinyl chloride, polyethylene and/or halogenated fluorocarbon.

A controlled release composition of the chemosensitising compounds described herein, may also be in the form of a buoyant tablet or capsule, i.e. a tablet or capsule which upon oral administration floats on top of the gastric content for a certain period of time. A buoyant tablet formulation of the chemosensitising compound in question can be prepared by granulating a mixture of the chemosensitising compound, excipients and 20-75% w/w of hydrocolloids, such as hydroxyethylcellulose, hydroxypropylcellulose and hydroxypropylmethylcellulose. The obtained granules can then be compressed into tablets. On contact with the gastric juice, the tablet can form a substantially water-impermeable gel barrier around its surface. This gel barrier takes part in maintaining a density of less than one, thereby allowing the tablet to remain buoyant in the gastric juice.

Powders, dispersible powders or granules suitable for preparation of an aqueous suspension by addition of water are also convenient dosage forms. Formulation as a suspension provides the chemosensitising compound in admixture with a dispersing or wetting agent, suspending agent and one or more preservatives.

Suitable dispersing or wetting agents are, for example, naturally-occurring phosphatides, as e.g. lecithin, or condensation products of ethylene oxide with e.g. a fatty acid, a long chain aliphatic alcohol or a partial ester derived from fatty acids and a hexitol or a hexitol anhydrides, for example, polyoxyethylene stearate, polyoxyethylene sorbitol monooleate, polyoxyethylene sorbitan monooleate ,etc.

Suitable suspending agents are, for example, sodium carboxymethylcellulose, methylcellulose, sodium alginate, etc.

The pharmaceutical composition may also be administered parenterally by injection, infusion or implantation (intravenous, intramuscular, intraarticular, subcutaneous or the like) in dosage forms, formulations or e.g. suitable delivery devices or implants containing conventional, non-toxic pharmaceutically acceptable carriers and adjuvants.

The formulation and preparation of such compositions is well-known to those skilled in the art of pharmaceutical formulation. Specific formulations can be found in the textbook entitled "Remington's Pharmaceutical Sciences".

Compositions for parenteral use may be presented in unit dosage forms, e.g. in ampoules, or in vials containing several doses and in which a suitable preservative may be added (see below). The composition may be in form of a solution, a suspension, an emulsion, an infusion device or a delivery device for implantation or it may be presented as a dry powder to be reconstituted with water or another suitable vehicle before use. Apart from the chemosensitising compounds described herein, the compositions may comprise suitable parenterally acceptable carriers and/or excipients or the active drug substance may be incorporated into microspheres, microcapsules, nanoparticles, liposomes or the like for controlled release. Furthermore, the composition may, in addition, conveniently comprise suspending, solubilising, stabilising, pH-adjusting agents and/or dispersing agents.

In another interesting embodiment of the invention, the pharmaceutical composition is a solid dosage form, such as a tablet, prepared from the particulate material described in WO 03/004001 and WO 2004/062643.

As indicated above, the pharmaceutical compositions may contain the chemosensitising compound in the form of a sterile injection. To prepare such a composition, the chemosensitising compound is dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution and isotonic sodium chloride solution. The aqueous formulation may also contain one or more preservatives, for example, methyl, ethyl or n-propyl p-hydroxybenzoate. In cases where chemosensitising compound is only sparingly or slightly soluble in water, a dissolution enhancing or solubilising agent can be added or the solvent may apart from water comprise 10-60% w/w of propylene glycol or the like.

<u>Dosages</u>

As discussed in detail previously, an important aspect of the present invention is the realisation that the chemosensitising compounds described herein are capable of reversing resistance or multidrug resistance when administered in clinical relevant amounts, i.e. in amounts sufficiently small to avoid the severe side effects normally associated with the chemosensitising compounds described herein.

It will be understood that the dosage to be administered will be dependent on the administration form (see below). Independently, of the administration form, the chemosensitising compound should be administered in clinically relevant amounts, i.e. in amounts which on the one hand exert the relevant therapeutic effect, but on the other hand does not provide severe side effects.

Preferably, a chemosensitising compound as described herein is administered in a clinically relevant amount giving rise to a steady state serum concentration of less than 8.0 mg/l. More preferably, the chemosensitising compound is administered in a clinically relevant amount giving rise to a steady state serum concentration of less than 7.0 mg/l, such as less than 6.0 mg/l, e.g. less than 5.0 mg/l. Even more preferably, the chemosensitising compound is administered in a clinically relevant amount giving rise to a steady state serum concentration of less than 4.0 mg/l, such as less than 3.0 mg/l, e.g. less than 2.0 mg/l. Most preferably, the chemosensitising compound is administered in a clinically relevant amount giving rise to a steady state serum concentration of less than 1.5 mg/l, e.g. about 1.0 mg/l or about 0.5 mg/l.

In other words, the chemosensitising compound is preferably administered in a clinically relevant amount giving rise to a steady state serum concentration in the interval of from 0.01 μg/l to less than 8.0 mg/l, such as in the interval of from 0.02 μg/l to 7.0 mg/l, e.g. in the interval of from 0.04 μg/l to 6.0 mg/l. More preferably, the steady state serum concentration of the chemosensitising compound is in the interval of from 0.06 μg/l to 5.0 mg/l, such as is in the interval of from 0.08 μg/l to 4.0 mg/l, e.g. in the interval of from 0.1 μg/l to 3.0 mg/l. Even more preferably, the steady state serum concentration of the chemosensitising compound is in the interval of from 0.2 μg/l to 2.0 mg/l, such as in the interval of from 0.4 μg/l to 2.0 mg/l, e.g. in the interval of from 0.5 μg/l to 2.0 mg/l. Still more preferably, the steady state serum concentration of the chemosensitising compound is in the interval of from 0.6 μg/l to 2.0 mg/l, such as in the interval of

from 0.8 μg/l to 2.0 mg/l, e.g. in the interval of from 0.9 μg/l to 2.0 mg/l. Most preferably, the steady state serum concentration of the chemosensitising compound is in the interval of from 1.0 μg/l to 2.0 mg/l, such as in the interval of from 1.5 μg/l to 2.0 mg/l, e.g. in the interval of from 1.5 μg/l to 1.5 mg/l.

The chemosensitising compound is preferably administered in an amount of about 0.1 to 3000 mg per day, such as about 0.5 to 2000 mg per day. As will be understood by the skilled person, the actual amount to be administered will *inter alia* be dependent on the administration route, i.e. whether the chemosensitising compound is administered orally, intravenous, intramuscular, etc.

For compositions adapted for oral administration for systemic use, the dosage is normally 1 mg to 3 g per dose administered 1-4 times daily for 1 day to 12 months depending on the infectious disease to be treated.

For parenteral administration, in particular intravenous administration, a dose of about 0.1 to about 2000 mg per day is convenient. For intravenous administration a dose of about 0.1 to about 2000 mg per day administered for 1 day to 12 months is convenient.

The above-mentioned steady state serum concentrations and dosages will give rise to the desired clinical effects and, at the same time, avoid the severe side effects normally associated with the chemosensitising compounds described herein. Some of the chemosensitising compounds described herein, in particular the chemosensitising compounds of the general formula IIIb, may however be administered in higher amounts, thereby giving rise to steady state serum concentrations above the levels indicated above. This is due to the fact that these chemosensitising compounds are expected not to exhibit severe side effects, even when administered in higher amounts.

The invention is further illustrated by the below, non-limiting, examples.

## MATERIALS AND METHODS

### Bacteria

Bacteria were sub-cultured on Mueller-Hinton agar plates and incubated over night at 37°C. Pre-warmed Mueller-Hinton broth was inoculated with colonies from the plate and incubated for approximately three hours to achieve a log phase culture. The log phase culture of bacteria was diluted with fresh pre-warmed medium and adjusted to a defined Optical Density (OD) at 600 nm in order to give a final assay concentration of 1 x $10^4$ bacteria/ ml medium.

The DR cells were approximately 10 to 100 times more resistant compared to sensitive cell lines and maintained a stable DR phenotype when grown in drug-free medium. The only exception was the *Enterococcus faecalis* F84 isolate. This strain was cultivated in the presence of vancomycin at 4 μg/ml in order to retain the MIC value at 4 μ/ml.

Strains were obtained from Statens Seruminstitut, Denmark, the Technical University of Denmark, and the Department of Clinical Microbiology, Soenderborg Hospital, Denmark.

### Isolates

*E. coli,* LN 3164. Acr AB-ToIC MDR *in vitro*-selected mutant. Resistant to tetracycline, beta-lactams, flouroquinolones, chloramphenicol and aminoglycosides.

*E. coli* 331 ME. *In vivo*-selected multidrug resistant clinical isolates of *E. coli.* Strains were isolated from a patient with severe cystitis/urosepsis. Resistant to tetracycline, beta-lactams, flouroquinolones, chloramphenicol and aminoglycosides.

*P. aeruginosa* 432b. Clinical multidrug resistant isolate. Resistant to tetracycline, beta-lactams, flouroquinolones, and aminoglycosides. Beta-lactamase producing, changes in Penicillin Binding Proteins and changes in Outer Membrane Proteins.

*Staphylococcus aureus* E45 MRSA. Clinical isolate. Resistant to methicillin. Susceptible to teicoplanin chloramphenicol, fosfomycin, netilmicin and vancomycin.

*Staphylococcus aureus* O11. Clinical isolate resistant to penicillin. Susceptible to methicillin, tetracycline, beta-lactams, flouroquinolones, chloramphenicol and aminoglycosides.

*Enterococcus faecalis,* F84. A multidrug resistant clinical isolate. Resistant to ampicillin, ciprofloxacin, gentamicin, and decreased resistance to vancomycin. Expressing change in the cell wall precurcer target as a major resistance mechanism (VanA gene expression).

*Enterococcus faecalis*, F86. Susceptible clinical isolate. No development of resistance.

### Drugs

Drugs were dissolved in small amounts of water or 1% DMSO (final culture concentration of DMSO less than 0.05% DMSO) before dilution with medium. Solutions were freshly prepared for each experiment.

Chlorpromazine, promazine, promethazine, prochlorperazine, trifluoperazine, fluphenazine, thioridazine, chlorpotixene, *trans*-clopenthixol, *cis*- and trans-flupenthixol were obtained from H. Lundbeck (Copenhagen, Denmark). 7-hydroxychlorpromazine, 7,8 dihydroxychlorpromazine, desmethylchlorpromazine hydrochloride, trifluopromazine, chlorpromazine sulfoxide, 1-chlorpromazine, phenothiazine, 2-chloro-10-(2-dimethylaminoethyl)-, hydrochloride were obtained from the National Institute of Mental Health (USA). Thiomethylpromazine was obtained from Statens Seruminstitut (Copenhagen Denmark). Perphenazine was obtained from Sigma (Copenhagen Denmark). Fucidic acid was obtained from Leo Pharma AS (Copenhagen Denmark), Aztreonam was obtained from Bristol Meyers (Bromma, Sweden). Vancomycin was obtained from Alpharma AS (Copenhagen Denmark). Ciprofloxacin was obtained from 1A Pharma (Copenhagen, Denmark), and gentamicin was obtained from Schering-Plough Europe (Brussels, Belgium).

**Effect of drugs on microbial cell growth and DR**

Cell growth was tested using the MIC susceptibility tests by use of the microdilution broth method in accordance to the NCCLS Guidelines (NCCLS Guidelines, Methods for Dilution Antimicrobial Susceptibility Tests for Bacteria That Grow Aerobically; Approved Standard, Sixth Edition, Volume 23; Number 2). A 100% Mueller-Hinton broth and a bacterial concentration of 1 x $10^4$/ml were used.

A log phase culture of bacteria was diluted with fresh pre-warmed medium and adjusted to a defined OD at 600 nm in order to give a final concentration of 1 x $10^4$ bacteria/ml medium in each well. Each chemosensitising compound was added to the bacterial culture in the wells in two-fold dilutions in order to give final concentrations ranging from 8 to 500 μg/ml. Trays were incubated at 37°C for 16 h. Minimal Inhibitory Concentration, (MIC) is defined as the highest lowest inhibitory concentration showing no visible growth according to the NCCLS Guidelines.

The effects of the chemosensitising compounds on DR were studied by the microtiter assay described above by exposing cells to 0-64 μg/ml anti-infective drug in the absence or presence of chemosensitising compound. Each experiment was repeated in tripleduplicate. MIC values represent the mean values of two separate experiments.

A log phase culture of bacteria was diluted with fresh pre-warmed medium and adjusted to a defined OD at 600 nm in order to give a final concentration of 1 x $10^4$ bacteria/ml medium in each well. A chemosensitising compound was added to the bacterial culture in the wells in order to give final concentrations at ¼ of the chemosensitising compound's MIC value. Anti-infective agent was added to the bacterial culture in the wells in two-fold dilutions in order to give final concentrations ranging from 0 to 16 μg/ml. Trays were incubated at 37°C for 16 h. The DR ratio was defined as the ratio between the MIC value for anti-infective agent alone divided by the MIC for the anti-infective agent in the presence of the chemosensitising compound. This ratio represents the increase in apparent potency of the anti-infective agent caused by the chemosensitising compound, and may be expressed as

$$\text{DR ratio} = (\text{MIC}_{\text{anti-infective agent}})/(\text{MIC}_{\text{anti-infective agent + chemosensitising compound}})$$

## EXAMPLES

### Example 1 - Effect of modifying promazine

Table 1 shows the structures, MIC values and DR Ratios for a series of promazine derivatives having different $R_1$, $R_2$, $R_7$ and $R_8$ substitutents.

The employed anti-infective agent was ciprofloxacin and the bacterial strain was *E. coli,* LN 3164.

The chemosensitising compounds (promazine and derivatives thereof) had the following general structure:

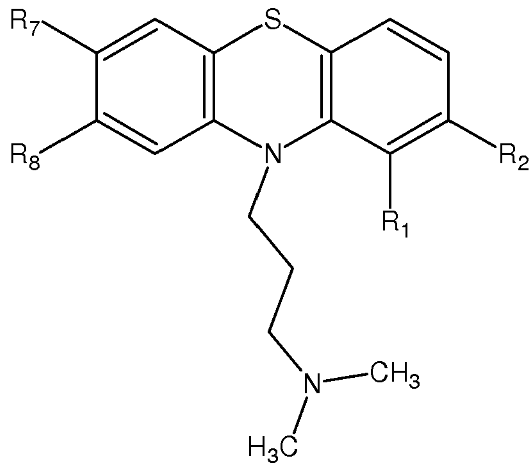

The obtained results are compiled in Table 1 below:

Table 1

| $R_1$ | $R_2$ | $R_7$ | $R_8$ | Name | MIC (μg/ml) | DR ratio |
|---|---|---|---|---|---|---|
| H | H | H | H | Promazine | 64 | 2 |
| Cl | H | H | H | 1-chlorpromazine | 64 | 2 |
| H | Cl | H | H | chlorpromazine | 32 | 4 |
| H | Cl | OH | H | 7-hydroxychlorpromazine | 125 | 1 |
| H | Cl | OH | OH | 7,8-dihydroxychlorpromazine | 125 | 0.5 |
| H | $S-CH_3$ | H | H | Thiomethylpromazine | 32 | 4 |
| H | $CF_3$ | H | H | Trifluopromazine | 32 | 8 |

As can be seen, the unsubstituted chemosensitising compound (promazine) inhibited cell growth and sensitised drug resistant *E. coli* cells to ciprofloxacin by 100% (DR ratio = 2). However, introduction of a chlorine atom at position 1 or 2 increased the potency against drug resistance. In particular, introducing the chlorine atom at position 2 had the greatest effect and sensitised the drug resistant cells by 220%. Similarly, introducing a $CF_3$ group at position 2 also increased potency against cell growth and drug resistance. Oxidation of the ring sulfur atom to produce chlorpromazine sulfoxide had the same activity against drug resistance as did chlorpromazine.

In order to determine the influence of the amino side chain, chemosensitising compounds having the following general structures were assayed as described above:

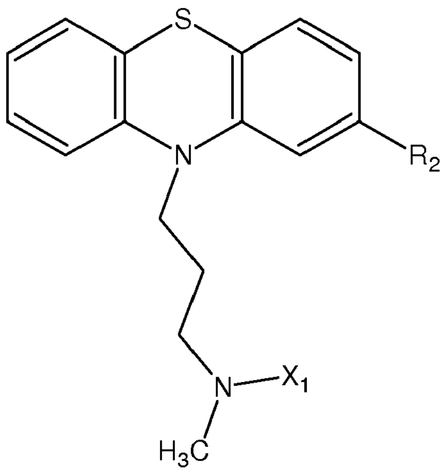

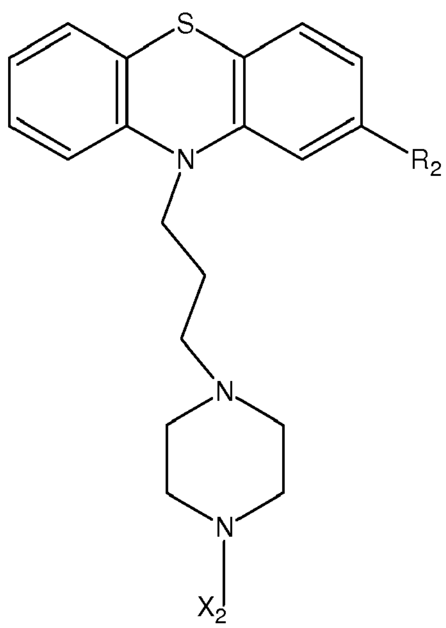

The obtained results are compiled in Table 2 below:

Table 2

| $R_2$ | $X_1$ | $X_2$ | Name | MIC (μg/ml) | DR ratio |
|---|---|---|---|---|---|
| Cl | H | - | Desmethylchlorpromazine | 32 | 2 |
| Cl | $CH_3$ | - | Chlorpromazine | 32 | 4 |
| $CF_3$ | $CH_3$ | - | Trifluopromazine | 32 | 8 |
| Cl | - | $CH_2$-$CH_2$-OH | Perphenazine | 125 | 4 |
| Cl | - | $CH_3$ | Prochlorperazine | 64 | 4 |
| $CF_3$ | - | $CH_2$-$CH_2$-OH | Fluphenazine | 32 | 8 |
| $CF_3$ | - | $CH_3$ | Trifluoperazine | 16 | 16 |

Table 2 above shows that phenothiazines containing tertiary amines (e.g. chlorpromazine) and secondary amines (desmethylchlorpromazine) possess similar activity in inhibiting cell growth (MICs of 32 μg/ml). However, phenothiazines containing tertiary amines, such as chlorpromazine, were more potent antagonists of DR than those with secondary amines, such as desmethylchlorpromazine, producing a 1.6 fold increase in anti-DR activity. Other changes in the type of amino group also affected anti DR activity. For example, piperazinyl derivatives increased potency against DR. Accordingly, the DR ratios for Trifluoperazine and Fluphenazine compounds were greater than that of Trifluopromazine, a compound with an identical ring subsitution pattern, but possessing an aliphatic side chain. Similarly Perphenazine and Prochlorperazine were more potent DR antagonists than chlorpromazine. This series of experiments also points out the importance of the $CF_3$ substitution at position 2 for anti DR activity. For example the DR ration for Trifluoperazine was greater than that for Prochlorperazine. These phenothiazines have identical structures except that the former has a $CF_3$ group instead of a chlorine atom at position 2. A similar relationship is seen by comparing fluphenazine to perphenazine. Finally a *para* methyl substitution on the piperazinyl ring appeared to be more potent than a *para* ethanol substitution, as can be seen by comparing the DR ratios for prochlorperazine to perphenazine or trifluoperazine to fluphenazine.

In order to determine the influence of the length of the amino-containing side chain, chemosensitising compounds having the following general structures were assayed as described above:

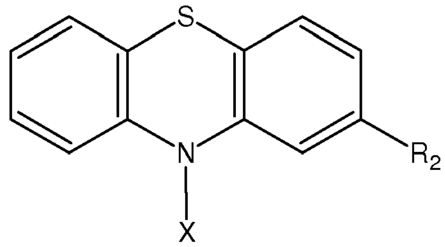

The obtained results are compiled in Table 3 below:

Table 3

| $R_2$ | X | Name | MIC ($\mu$g/ml) | DR ratio |
|---|---|---|---|---|
| Cl | $CH_2$-$CH_2$-$N(CH_3)_2$ | 2-chloro-10-dimethylamino-ethyl)phenothiazine | 64 | 2 |
| Cl | $(CH_2)_3$-$N(CH_3)_2$ | Chlorpromazine | 32 | 4 |
| Cl | $CH_2$-$CH(CH_3)$-$N(CH_3)_2$ | Promethazine | 64 | 8 |
| H | $CH_2$-$CH_2$-$N(CH_2)$ | Promazine | 64 | 2 |

Table 3 above shows the effect on cell growth and DR of a series of dimethylamino-phenothiazines in which the length of the amino-containing side chain was varied. As can be seen, moving from a two to three carbon alkyl bridge increased the anti DR effects of these chemosensitising compounds. For example, Chlorpromazine had a greater DR ratio than that of 2-chloro-10-(2-dimethylaminoethyl)phenothiazine. Promethazine, which has an isopropyl side chain, was a more potent DR inhibitor compared to promazine, which has a straight three-carbon alkyl chain.

**Example 2 - Effect of stereochemistry**

In order to investigate the influence of the cis and trans stereochemistry a series of thioxanthenes were assayed as described above. Table 4 shows the MIC values and DR Ratios for the tested thioxanthenes.

Table 4

| Name | MIC ($\mu$g/ml) | DR ratio |
|---|---|---|
| trans-flupenthixol | 32 | 64 |
| cis-flupenthixol | 32 | 32 |
| *trans*-clopenthixol | 16 | 128 |

The above results demonstrate that stereoisomeric configurations are required for optimum activity against DR. For example, trans-flupenthixol is a more potent anti-DR agent than the cis-form of the compound and *trans*-clopenthixol was the most potent anti-DR agent. Thus, the orientation of the side chain amine in relation to the tricyclic nucleus appears to be an important determinant for anti-DR activity.

**Example 3 - Effect of hydrophobicity**

To determine if the differences in anti DR potency in chemosensitising compounds with side chain alterations were also due to changes in overall hydrophobicity, the octanol:buffer partition coefficients for each of the drugs in Tables 1, 2, 3 and 4 were compared to their DR ratios. No statistically significant correlation was found between hydrophobicity and anti DR activity ($p>0.5$) (Data not shown).

**Example 4 - Synergistic effects of thioxanthene derivatives**

The synergistic effect of trans-flupenthixol, cis-flupenthixol and *trans*-clopenthixol on a variety of anti-infective agents was examined against a panel of susceptible and resistant bacterial isolates representing different species and resistance mechanisms.

The synergistic effect was studied by checkerboard combination studies exposing cells to 0-16 μg/ml anti-infective agent in the absence or presence of trans-flupenthixol, *cis*-flupenthixol or *trans*-clopenthixol. Each experiment was repeated in tripleduplicate. MIC values represent the mean values of two separate experiments,

A log phase culture of bacteria was diluted with fresh pre-warmed medium and adjusted to a defined OD at 600 nm in order to give a final concentration of 1 x $10^4$ bacteria/ml medium in each well. Each chemosensitising compound was added to the bacterial culture in the wells in two-fold dilutions in order to give final concentrations ranging from 0 to 64 μg/ml. Anti-infective agent was added to the bacterial culture in the wells in two-fold dilutions in order to give final concentrations ranging from 0 to 16 μg/ml Trays were incubated at 37°C for 16 h. Wells were assessed visually for growth. The Fractional Inhibitory Concentration (FIC) was calculated for each anti-infective agent alone and in combination with trans-flupenthixol, *cis*-flupenthixol or *trans*-clopenthixol. The following formulae were used to calculate the FIC index:

$$FIC = FIC_{\text{chemosensitising compound}} + FIC_{\text{anti-infective agent}}$$

where:

$$FIC_{\text{chemosensitising compound}} = (MIC_{\text{chemosensitising compound + anti-infective agent}})/(MIC_{\text{chemosensitising compound}})$$

$$FIC_{\text{anti-infective agent}} = (MIC_{\text{anti-infective agent + chemosensitising compound}}/(MIC_{\text{anti-infective agent}})$$

Synergy was defined as a FIC index of <0.5. The calculated FIC indices are shown in Table 5 below:

Table 5. *R=Isolate resistant to anti-infective agent; S=isolate susceptible to anti-infective agent; MDR= multidrug resistant isolate.

| Isolate | Anti-infective agent | Resistance* | cis-flupenthixol | *trans*-flupenthixol | *trans*-clopenthixol |
|---|---|---|---|---|---|
| E. faecalis F84 | Vanomycin | MDR | 0.25 | 0.13 | 0.06 |
| | Dicloxacillin | | 0.13 | 0.06 | 0.03 |
| E. faecalis F86 | Dicloxacillin | S | 0.50 | 0.50 | 0.25 |
| S. aureus E45 | Dicloxacillin | R | 0.25 | 0.13 | 0.06 |
| | Fucidic acid | R | 0.26 | 0.13 | 0.06 |
| S. aureus O11 | Dicloxacillin | S | 0.50 | 0.50 | 0.25 |
| E. coli LN 3164 | Ciprofloxacin | MDR | 0.25 | 0.13 | 0.063 |
| | Gentamicin | | 0.25 | 0.063 | 0.063 |
| | Piperacillin | | 0.25 | 0.13 | 0.063 |
| E. coli 331 ME | Ciprofloxacin | MDR | 0.013 | 0.006 | 0.003 |
| | Gentamicin | | 0.06 | 0.02 | 0.008 |
| | Piperacillin | | 0.25 | 0.13 | 0.063 |
| P. aeruginosa | Ciprofloxacin | R | 0.06 | 0.03 | 0.008 |
| | Gentamicin | R | 0.13 | 0.06 | 0.032 |
| | Piperacillin | R | 0.25 | 0.13 | 0.063 |

The FIC indices for *trans*-clopenthixol and *cis*- and *trans*-flupenthixol show that these compounds are strongly synergistic in promoting the bacteriostatic effects of the anti-infective agents in the drug resistant cells. Most of the FIC indices for the chemosensitising compounds assayed on drug-resistant cells were <<0.5. *Trans*-clopenthixol was the most potent of all the tested chemosensitising compounds, i.e. the *trans* form was more potent compared to the *cis* form.

Thus the clinical use of e.g. *trans*-clopenthixol or *trans*-flupenthixol in combination with an anti-infective agent would likely shift the MIC of this anti-infective agent for the DR cells to well-below the clinically achievable concentration, showing effective concentrations at <5 ng/ml. The anti DR effect was most potent in resistant cells. However a remarkable antibiotic enhancing effect was shown also in the susceptible cells strongly indicating that the anti DR effect of these chemosensitising compounds is not restricted to cells overexpressing efflux pumps and the anti DR mechanism is not restricted to this target. FIC indices for antibiotic susceptible cells ranged from 0.25 to 0.5.

Furthermore, the results demonstrate that *cis*- and trans-flupenthixol as well as *trans*-clopenthixol enhanced the anti-infective activity of the beta-lactam antibioticum piperacillin against *P.aeruginosa* cells expressing beta-lactamase able to inhibit piperacillin (Giwercman B et al, 1992 and 1990) suggesting that the anti DR mechanism of the compounds is not restricted to inhibition of MDR efflux pumps. The antiproliferative effects of the chemosensitising compounds were approximately equipotent modest in both the sensitive and resistant isolates. MIC values ranged from 16 to 64 μg /ml (data not shown).

**Example 5 - Development of insensitivity to the chemosensitising compounds**

One potential limitation to the combination of an anti-infective agent with inhibitors of resistance mechanism (s) is the possibility of the microorganism develops mutations which render it insensitive to the inhibitor. Such a situation has been observed for e.g. bacteria, virus, fungi and yeast.

The effect of the inhibitors on the rate of emergence of *in vitro*-selected single-step ciprofloxacin resistance on the clinical isolate of S. *aureus* O11 was determined.

Spontaneous mutants were obtained 24 h after plating S. *aureus* cells on LB agar plates containing ciprofloxacin at a concentration of 1 μg/ml (two times the MIC) in the absence or presence of *trans*-clopenthixol at 1 μg/ml. The frequency of mutant selection was determined to be $3\times 10^{-8}$ by comparing the number of colonies that grew on plates containing the anti-infective agent with the number of colonies obtained upon plating appropriate dilutions in the absence of anti-infective agents.

The probably most important aspect, when assessing the use of the inhibitors in the clinic, is the effect of these inhibitors on the emergence of resistant mutants. Importantly, and as shown in Table 6, the tested inhibitor decreased the frequency of spontaneous emergence of ciprofloxacin resistance by 100-fold or more. This dramatic effect could not be attributed to a toxic effect of the inhibitor since the same concentration of inhibitor, which was at least 1 0-fold less than its MIC for S. *aureus,* affected neither the colony-forming ability nor the colony size of S. *aureus* cells plated in the absence of ciprofloxacin. In conclusion, the trans-clopenthixol inhibited the emergence of ciprofloxacin resistance in S. *aureus*.

| Inhibitor (1 μg/ml) | Frequency on emergence of resistance |
|---|---|
| None | $3 \times 10^{-8}$ |
| *trans*-clopenthixol | $<1 \times 10^{-10}$ |

Table 6. Frequency of emergence of *in vitro*-selected variants of S. *aureus* resistant to 1 μg of ciprofloxacin per ml (two times the MIC for the S. *aureus* strain) in either the absence or the presence of inhibitors.

## Claims

1. Compound of the general formula (I)

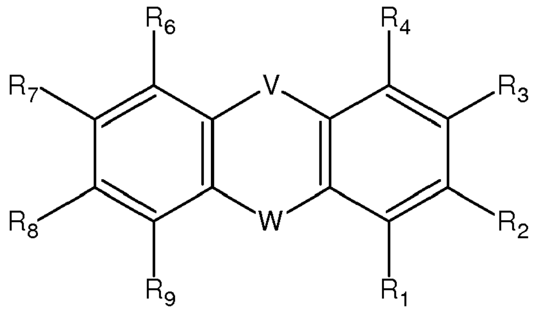

(I)

wherein
V is S;
W is $C=CH-(CHX)_m-N(R_{10})(R_{11})$;
m is an integer in the range of from 1 to 5;
each X is individually selected from the group consisting of hydrogen, halogen, hydroxy, amino, nitro, optionally substituted $C_{1-6}$-alkyl and optionally substituted $C_{1-6}$-alkoxy;
$R_2$ is selected from the group consisting of F, Cl, Br, I, $CH_2Y$, $CHY_2$ and $CY_3$, wherein Y is a halogen atom;
$R_1$, $R_3$, $R_4$, $R_6$, $R_7$, $R_8$ and $R_9$ are each individually selected from the group consisting of hydrogen, halogen, hydroxy, amino, nitro, optionally substituted $C_{1-6}$-alkyl, optionally substituted $C_{2-6}$-alkenyl, optionally substituted $C_{2-6}$-alkynyl and optionally substituted $C_{1-6}$-alkoxy, optionally substituted $C_{2-6}$-alkenyloxy, carboxy, optionally substituted $C_{1-6}$-alkoxycarbonyl, optionally substituted $C_{1-6}$-alkylcarbonyl, fomyl, optionally substituted $C_{1-6}$-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, mono- and di($C_{1-6}$-alkyl)amino, carbamoyl, mono- and di($C_{1-6}$-alkyl)aminocarbonyl, amino-$C_{1-6}$-alkyl-aminocarbonyl, mono- and di($C_{1-6}$-alkyl)amino-$C_{1-6}$-alkyl-aminocarbonyl, $C_{1-6}$-alkylcarbonylamino, amino-$C_{1-6}$-alkyl-carbonylamino, mono- and di($C_{1-6}$-alkyl)amino-$C_{1-6}$-alkyl-carbonylamino, amino-$C_{1-6}$-alkyl-amino, mono- and di($C_{1-6}$-alkyl)amino-$C_{1-6}$-alkyl-amino, cyano, guanidino, carbamido, $C_{1-6}$-alkanoyloxy, $C_{1-6}$-alkylsulphonyl, $C_{1-6}$-alkylsulphinyl, $C_{1-6}$-alkylsulphonyloxy, aminosulfonyl, mono- and di($C_{1-6}$-alkyl)aminosulfonyl, and optionally substituted $C_{1-6}$-alkylthio; and
$R_{10}$ and $R_{11}$ are each independently selected from the group consisting of hydrogen, optionally substituted $C_{1-6}$-alkyl, optionally substituted $C_{2-6}$-alkenyl, optionally substituted $C_{2-6}$-alkynyl, optionally substituted $C_{1-6}$-alkoxycarbonyl, optionally substituted $C_{1-6}$-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di($C_{1-6}$-alkyl)aminocarbonyl; or $R_{10}$ and $R_{11}$ together with the nitrogen atom to which they are attached form an optionally substituted nitrogen-containing heteroaryl or optionally substituted heterocyclyl;
or a salt thereof for use in combination with an anti-infective agent for the treatment or prophylaxis of an infectious disease caused by a drug resistant infectious agent.

2. Compound according to claim 1, wherein $R_2$ is selected from the group consisting of F, Cl, $CF_3$ and $CCl_3$.

3. Compound according to claim 2, wherein $R_2$ is Cl or $CF_3$.

4. Compound according to any of the preceding claims, wherein $R_1$, $R_3$, $R_4$, $R_6$, $R_7$, $R_8$ and $R_9$ are each individually selected from the group consisting of hydrogen, optionally substituted $C_{1-6}$-alkyl and optionally substituted $C_{1-6}$-alkoxy.

5. Compound according to claim 4, wherein $R_1$, $R_3$, $R_4$, $R_6$, $R_7$, $R_8$ and $R_9$ are hydrogen.

6. Compound according to any of claims 1-5, wherein m is 2 or 3.

**7.** Compound according to claim 6, wherein m is 2.

**8.** Compound according to any of claims 1-7, wherein $R_{10}$ and $R_{11}$, together with the nitrogen atom to which they are attached, form an optionally substituted heterocyclyl.

**9.** Compound according to claim 8, wherein said optionally substituted heterocyclyl is selected from the group consisting of optionally substituted 2-pyrrolinyl, optionally substituted 3-pyrrolinyl, optionally substituted pyrrolidinyl, optionally substituted 2-imidazolinyl, optionally substituted imidazolidinyl, optionally substituted 2-pyrazolinyl, optionally substituted 3-pyrazolinyl, optionally substituted pyrazolidinyl, optionally substituted piperidinyl, optionally substituted morpholinyl, optionally substituted thiomorpholinyl and optionally substituted piperazinyl,

**10.** Compound according to claim 9, wherein said optionally substituted heterocyclyl is an optionally substituted piperidinyl or an optionally substituted piperazinyl.

**11.** Compound according to claim 10, wherein said piperazinyl is unsubstituted.

**12.** Compound according to any of the preceding claims, wherein said $C=CH-(CHX)_m-N(R_{10})(R_{11})$ group is in the *trans* configuration.

**13.** Compound according to any of the preceding claims, wherein said compound is administered in a clinically relevant amount giving rise to a steady state serum concentration of less than 8.0 mg/l.

**14.** Compound according to any of the preceding claims, wherein said infectious agent is multidrug resistant.

**15.** A pharmaceutical composition comprising a compound as defined in any of claims 1-14 and an anti-infective agent and at least one pharmaceutically acceptable carrier or excipient.

FIG.1

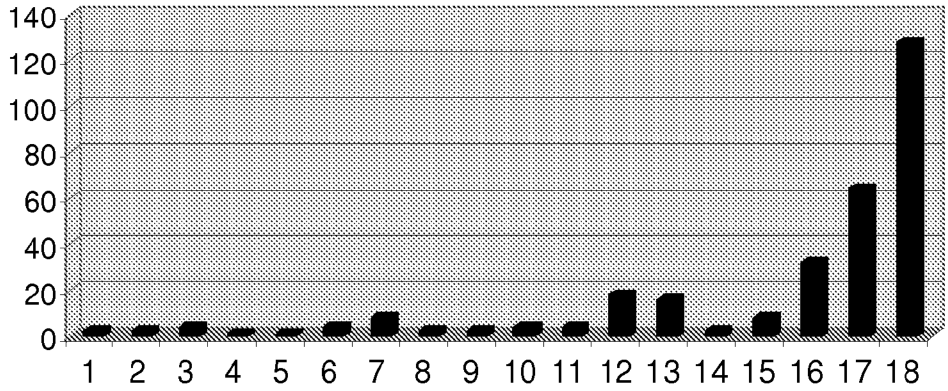

Fig. 2A E. coli

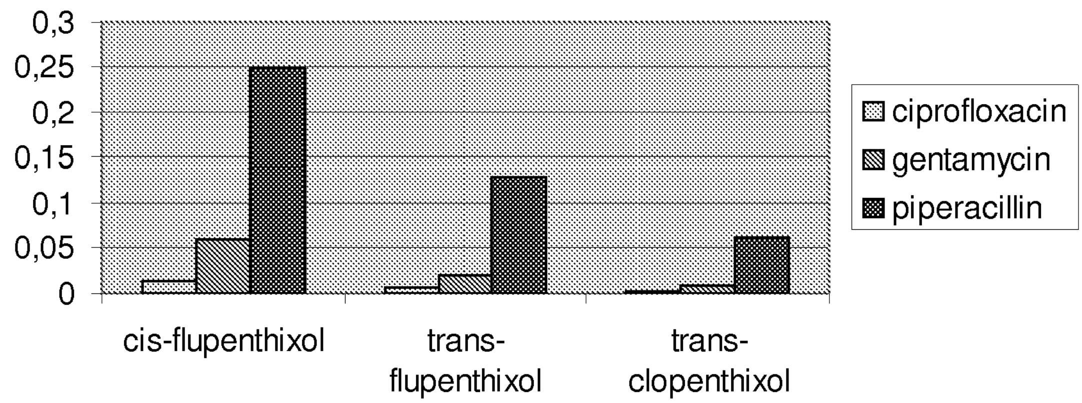


Fig. 2B Pseudomonas aeruginosa

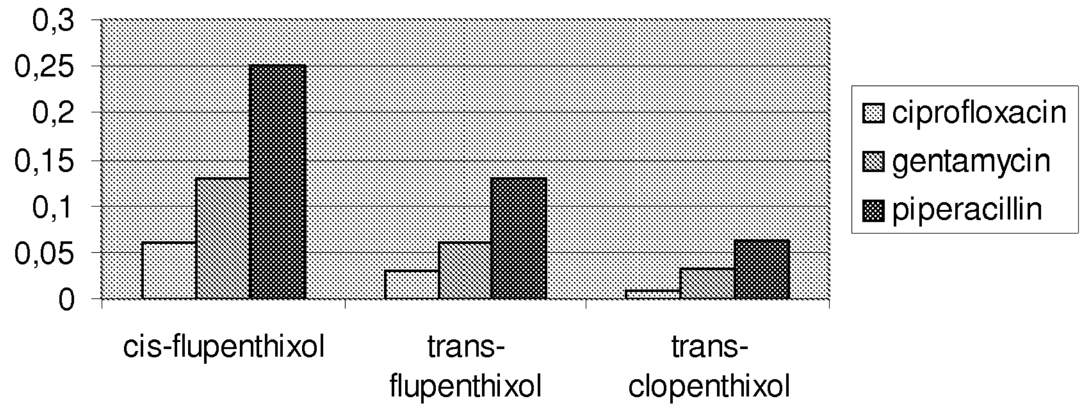

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6569853 B
- GB 925538 A
- GB 863699 A
- WO 03004001 A
- WO 2004062643 A

### Non-patent literature cited in the description

- Remington's - The Science and Practice of Pharmacy. Lippincott, Williams & Wilkins, 2000
- Remington's Pharmaceutical Sciences'' and ''Encyclopedia of Pharmaceutical Technology. Marcel Dekker, Inc, 1988
- Aqueous film coating. **James A. Seitz.** Encyclopedia of Pharmaceutical Technology. Marcel Dekker, Inc, 1988, vol. 1, 337-349